# EUROPÄISCHE PATENTSCHRIFT

(11) **EP 4 189 365 B1**
(45) Veröffentlichungstag und Bekanntmachung des Hinweises auf die Patenterteilung: **10.07.2024**
(21) Anmeldenummer: 21743525.4
(22) Anmeldetag: 21.07.2021
(51) Int. Cl.: G01N 21/17

(54) **VERFAHREN UND SENSOR ZUR DETEKTION VON AEROSOLPARTIKELN IN EINER UMGEBUNGSLUFT**
METHOD AND SENSOR FOR DETECTING AEROSOL PARTICLES IN AMBIENT AIR
PROCÉDÉ ET CAPTEUR DE DÉTECTION DES PARTICULES D'AÉROSOL DANS UN AIR AMBIANT

(30) Priorität: 29.07.2020 EP 20188316
(43) Veröffentlichungstag der Anmeldung: 07.06.2023
(73) Patentinhaber: Hahn-Schickard-Gesellschaft für angewandte Forschung e. V., 70569 Stuttgart (DE)
(72) Erfinder: BITTNER, Achim, 74080 Heilbronn (DE); DEHÉ, Alfons, 72770 Reutlingen (DE); WIENBRUCH, Rebecca, 78267 Aach (DE)
(74) Vertreter: Hertin und Partner Rechts- und Patentanwälte PartG mbB
(86) Internationale Anmeldenummer: PCT/EP2021/070394
(87) Internationale Veröffentlichungsnummer: WO 2022/023141

(56) Entgegenhaltungen:
- WO-A1-2015/020611
- DE-A1- 4 130 639
- US-A1- 2003 159 498
- US-B2- 9 964 470

## Beschreibung

Die Erfindung betrifft in einem ersten Aspekt ein Verfahren zur Detektion von Aerosolpartikeln in einer Umgebungsluft mittels eines photoakustischen Gassensors, wobei ein Analysevolumen im Strahlengang eines modulierbaren Emitters vorliegt, sodass der Emitter mittels modulierbarer Strahlung Aerosolpartikel in dem Analysevolumen zur Ausbildung von Schalldruckwellen anregen kann, welche mit Hilfe des Sensors detektierbar sind. Mit Hilfe des modulierbaren Emitters wird das Analysevolumen mit der modulierten Strahlung zur Erzeugung von Schalldruckwellen bestrahlt. Eine Messung der erzeugten Schalldruckwellen wird mittels des Sensors durchgeführt, wobei eine Feststellung der Präsenz und/oder Konzentration der Aerosolpartikel in der Umgebungsluft anhand der Messergebnisse erfolgt. Es handelt sich bei den Aerosolpartikeln um Bioaerosole, bevorzugt Pollen, Sporen, Bakterien oder Viren. In einem weiteren Aspekt betrifft die Erfindung ein für die Durchführung des Verfahrens geeigneten photoakustischen Gassensor.

### Hintergrund und Stand der Technik

Die Erfindung betrifft das Gebiet der Detektion von biologischen Aerosolpartikeln in einer Umgebungsluft wie Bakterien, Sporen oder Viren, wie z.B. in US 2003/0159498 A1 offenbart.

Weltweit sind Infektionskrankheiten eine der häufigsten Todesursachen. Dabei entfallen die meisten Todesfälle auf Lungenentzündungen, Durchfallerkrankungen, AIDS, Tuberkulose und Malaria.

Die Behandlung von Infektionskrankheiten wird durch den Anstieg an Erregern, die gegen Arzneimittel (Antibiotika, Virostatika) resistent sind, zunehmend erschwert. Hauptursachen der zunehmenden Resistenzentwicklung sind der unsachgerechte Einsatz von Antibiotika und die inkonsequente Anwendung von notwendigen Hygienemaßnahmen zur Prävention von Infektionen.

Zudem zeigt der Ausbruch der Covid 19 Pandemie im Jahre 2020 eindrücklich, dass neu auftretende Infektionskrankheiten, für welche weder Therapien noch ein Impfschutz zur Verfügung stehen, die menschliche Gesellschaften in ihren Grundfesten bedrohen können. Aerogene Infektionskrankheiten stellen unter den Infektionskrankheiten ein besonderes Risiko dar. Als aerogene Infektionskrankheiten bezeichnet man Infektionskrankheiten, die über den Luftweg (aerogen) durch Einatmung erregerhaltiger Schwebstoffe übertragen werden. Zu den aerogenen Infektionskrankheiten gehören beispielsweise die Tuberkulose, Pollen, Masern, Windpocken, Influenza oder Covid 19.

Weltweit erkranken allein jährlich über 10 Millionen Menschen pro Jahr an Tuberkulose, wovon durchschnittlich 1,5 Millionen Menschen versterben (Word Health Organisation WHO, Global tuberculosis report 2019*).* An Covid 19 haben sich weltweit allein in den Monaten von März bis Juli 2020 über 15 Millionen Menschen nachweislich infiziert, über 600 000 Todesfälle wurden verzeichnet (Quelle: WHO).

Die Schwierigkeit der Eindämmung aerogener Infektionskrankheiten und deren hohe Infektiosität hängt eng mit deren Ausbreitung zusammen. Eine Übertragung aerogener Infektionskrankheiten bedarf keinen unmittelbaren Kontakt zwischen Menschen, sondern kann mittelbar bzw. indirekt über sich in der Umgebungsluft ausbreitende Aerosole erfolgen.

Luftgetragene Partikel können auch noch einige Zeit nach einer anfänglichen Aerosolisierung in der Luft verbleiben und setzen daher potenziell eine viel höhere Anzahl von empfänglichen Individuen einer Infektionsgefahr aus. Abhängig von Umweltfaktoren (z.B. meteorologische Bedingungen im Freien, Druckunterschiede in Innenräumen etc.) können luftgetragene Partikel leicht über viele Meter transportiert und auch mehrere Stunden in Räumen verbleiben (Fernstrom A. et al 2013).

Eine unmittelbare Bestimmung der Erregerlast in der Umgebungsluft kann daher eine wichtige Säule in der Verhinderung oder Eindämmung von aerogenen Infektionskrankheiten darstellen. Auf Basis einer Messung der Erregerlast können beispielsweise Frühwarnsysteme innerhalb privater oder öffentlicher Gebäude etabliert werden und Infektionsketten wirksam unterbinden.

Bekannte Techniken zur Detektion von Bakterien oder Viruspartikeln in der Luft sind oftmals zeitintensiv oder erfordern aufwändige Instrumentation bzw. Probenmanipulationen (Antikörperbindung, elektrochemische Reaktionen).

Klassische Ansätze nutzen Sammelvorrichtungen für die Umgebungsluft. Bei diesen werden unter Verwendung geeigneter Substrate die Viren oder Bakterien immobilisiert. Eine molekularbiologische Identifikation der Erreger kann beispielsweise mittels einer Polymerasen-Ketten-Reaktion erfolgen (vgl. u.a. Schafer et al 1999 für die Detektion luftgetragener Tuberkelbazillen.)

Tobias et al. 2005 schlagen die Verwendung von Massenspektroskopie zur Detektion von aerogenen Tuberkulose-Bakterien vor. Senguptaa, A 2007 nutzen eine *surface-enhanced* Raman Spektroskopie zur Detektion von Bioaerosolen. Hierzu wurde eine Instrumentation entwickelt, um luftgetragene Pollen und Bakterien nachzuweisen und zu charakterisieren, indem ein Bioaerosol mit Hilfe einer Mikropumpe in eine nanokolloidale Suspension von Silberpartikeln injiziert wird. Die biologischen Partikel werden mit dem Silberkolloid gemischt, um die metallischen Partikel auf der Oberfläche des Bioanalytes abzulagern und anschließend deren Spektren zu messen. Aus Kyu-Tae Park et al 2015 ist ein Ansatz zur Detektion bekannt, welcher auf einer elektroaerodynamischen Deposition und einem Feld-Effekt-Transistor beruht.

Darüber hinaus gibt es auch eine Reihe von fluoreszenzbasierten Ansätzen zur Detektion von biologischen Partikeln in Aerosolen. Hierbei wird typischerweise monochromatisches Licht (kontinuierlich oder gepulst) genutzt, um die Fluoreszenzeigenschaften einzelner Partikel zu untersuchen, die in der Luft durch das Gerät strömen. Für die Detektion biologischer Moleküle, kann ausgenutzt werden, dass die Autofluoreszenz, d.h. die intrinsische Fluoreszenz von einigen Biomolekülen auf das Vorhandensein von biologischem Material hinweisen kann, während die Intensität der meisten nicht-biologischen Aerosole gering ist. Ähnlichkeiten in der spektralen Emission verschiedener biologischer Partikel führen jedoch zu einer verringerten Spezifizität (Huffmann et al. 2020).

Im Lichte der bekannten Techniken gibt es somit einen Bedarf an Verbesserung, welcher insbesondere den Aufwand der Instrumentation oder Probenmanipulationen verringert und dennoch zuverlässig eine Detektion insbesondere von Viren oder Bakterien in der Umgebungsluft erlaubt.

Die photoakustische Spektroskopie (PAS) ist eine etablierte Technik zur Detektion feinster Konzentrationen von Gasen und hat eine Vielzahl von Anwendungen. Beispielhaft ist die Detektion von CO₂, welche in der Forschung und der Klimatechnik eine Rolle spielt. Auch die Konzentration z. B. von Abgasen in der Luft kann so gemessen werden. Ebenso sind militärische Anwendungen relevant, bei denen kleinste Konzentrationen von Giftgas detektiert werden können.

Bei der photoakustischen Spektroskopie wird intensitätsmodulierte Infrarotstrahlung mit Frequenzen im Absorptionsspektrum eines in einem Gas zu detektierenden Moleküls eingesetzt. Ist dieses Molekül im Strahlengang vorhanden, findet eine modulierte Absorption statt, die zu Erwärmungs- und Abkühlungsprozessen führt, deren Zeitskalen die Modulationsfrequenz der Strahlung widerspiegeln. Die Erwärmungs- und Abkühlungsprozesse führen zu Expansionen und Kontraktion des Gases, wodurch Schallwellen mit der Modulationsfrequenz verursacht werden. Diese lassen sich dann durch Schalldetektoren, wie beispielsweise Mikrofone, oder Flusssensoren messen.

Die Verwendung von PAS zur spezifischen Detektion von Viren oder Bakterien innerhalb von Aerosolen ist weitestgehend unerforscht.

Die US 7710566 B2 PAS schlägt vor PAS zur Detektion von Staubpartikeln zu verwenden. Eine Detektion von Viren oder Bakterien wird nicht beschrieben. Auch ist der Geräteaufwand hoch.

Lack et al. 2007 ist ein Forschungsartikel zur photoakustischen Spektroskopie von Aerosolen. Ziel ist es mittels der photoakustischen Spektroskopie die Absorptionseigenschaften von Aerosolen im Hinblick auf die Klimaerwärmung zu untersuchen. Eine Detektion von Viren oder Bakterien mittels PAS wird nicht offenbart.

Die WO 2015/020611 A1 schlägt eine Vorrichtung und ein Verfahren zur Detektion von Aerosolpartikeln vor. Über einen Einlass gelangt ein Fluid in eine Messkammer, welche durch eine Strahlungsquelle bestrahlt wird. Die Vorrichtung umfasst einen MEMS-Resonator, welcher die Aerosolpartikel basierend auf deren Absorptionseigenschaften über bevorzugt zwei Moden, einer photoakustischen Mode und einer *bulk acoustic wave* (BAW) Mode detektieren soll. In der photoakustischen Mode wird der photoakustische Effekt oder der thermoakustische Effekt zur Detektion von Aerosolpartikeln genutzt. Der MEMS-Resonator detektiert eine Schalldruckwelle, die durch die Lichtabsorption der Aerosolpartikel hervorgerufen wird. Bei den Aerosolen handelt es sich um Staub- oder Rußpartikel. Die Verwendung einer photoakustischen Spektroskopie zur Detektion von Bioaerosolen, wie z.B. Viren, Bakterien oder Pollen wird nicht offenbart.

DE 41 30 639 A1 betrifft ein Gerät zur photoakustischen Detektion von Graphit- und Rußteilchen. Über eine Gaspumpe gelangen die Aerosole in eine resonante photoakustische Zelle, welche mit einem modulierbaren Halbleiterdiodenlaser bestrahlt wird. Bevorzugt wird hierbei nicht die erste Resonanzmode, sondern die Resonanzfrequenzen der nächsthöheren Mode zur Modulation verwendet, um niederfrequente Störeinflüsse zu vermeiden. Die DE 41 30 639 A1 hat das Ziel ein kompaktes und mobiles Rußdetektionssystem mit einer hohen Nachweisgrenze bereitzustellen. Die Detektion von Bioaerosolen, insbesondere von Pollen, Sporen, Bakterien oder Viren, wird auch in DE 41 30 639 A1 nicht offenbart. US 9 964 470 B2 offenbart ein Verfahren und ein System, um die Luftqualität eines Raumes zu kontrollieren. Das Verfahren nutzt Luftverunreinigungssensoren (*air containment sensor*), welche Teil eines *multipoint air monitoring* Systems sind. Mittels einer verbesserten Approximation eines *true indoor air containement levels* soll eine präzisere Einstellung einer notwendigen Ventilation und Luftzufuhr ermöglicht werden. Die *air containment sensors* werden nicht im Detail beschrieben.

In der US 9 964 470 B2 werden ein Vielzahl von Messverfahren aufgelistet, wobei ein photoakustisches Messprinzip neben einer Reihe weiterer Messverfahren (elektrochemisch, optisch etc.) genannt wird. Zu den zu überwachenden *air containments* sollen verschiedene Partikel gehören, wobei auch Bioaerosole wie z.B. Viren, Bakterien etc. genannt werden.

Eine unmittelbare Lehre zur Verwendung eines photoakustischen Messprinzips zur Detektion von Bioaerosolen, wie Pilzen, Viren oder Bakterien umfasst die US 9 964 470 B2 nicht. Vielmehr würde ein Fachmann im Hinblick auf den bisherigen Stand der Technik davon ausgehen, dass für die Detektion von Pilzen, Viren oder Bakterien beispielsweise elektrochemische Verfahren eingesetzt werden, welche in der Auflistung möglicher Messverfahren ebenfalls genannt werden. Ein Hinweis, dass ein photoakustische Messprinzip zur Detektion von Bioaerosolen genutzt werden kann, findet ein Fachmann in der US 9 964 470 B2 nicht.

Im Lichte des Standes der Technik gibt es somit einen Bedarf an verbesserten oder alternativen Verfahren oder Vorrichtungen zur Detektion von Aerosolpartikeln in einer Umgebungsluft.

### Aufgabe der Erfindung

Aufgabe der Erfindung ist es, ein Verfahren sowie eine hierzu geeignete Vorrichtung zur Detektion von Aerosolpartikeln in einer Umgebungsluft ohne die Nachteile des Standes bereitzustellen. Insbesondere war es eine Aufgabe der Erfindung, ein verbessertes Verfahren zur Verfügung zu stellen, welches zuverlässig, schnell, kostengünstig und ohne aufwändige Probenmanipulation oder Instrumentation Bioaerosolpartikel vermessen kann.

### Zusammenfassung der Erfindung

Gelöst wird die Aufgabe durch die Merkmale der unabhängigen Ansprüche. Vorteilhafte Ausgestaltungen der Erfindung sind in den abhängigen Ansprüchen beschrieben.

In einem Aspekt und gemäß Anspruch 1 betrifft die Erfindung ein Verfahren zur Detektion von Aerosolpartikeln in einer Umgebungsluft, umfassend die Schritte
a. Bereitstellung eines photoakustischen Gassensors, umfassend
   - einen modulierbaren Emitter, insbesondere eines MEMS-Emitters
   - ein Analysevolumen, welches in Fluidkommunikation mit der Umgebungsluft steht
   - einen Sensor zur Detektion von Schalldruckwellen, insbesondere einen MEMS-Sensor
   wobei das Analysevolumen im Strahlengang des Emitters vorliegt, sodass der Emitter mittels modulierbarer Strahlung Aerosolpartikeln in dem Analysevolumen zur Ausbildung von Schalldruckwellen anregen kann, welche mit Hilfe des Sensors detektierbar sind,
b. Bestrahlung des Analysevolumen mit einer mit einer Modulationsfrequenz modulierten Strahlung zur Erzeugung von Schalldruckwellen
c. Messung der erzeugten Schalldruckwellen mittels des Sensors
d. Feststellung der Präsenz und/oder Konzentration der Aerosolpartikeln in der Umgebungsluft anhand der Messergebnisse
wobei es sich bei den Aerosolpartikeln bevorzugt um Bioaerosole handelt.

Das erfindungsgemäße Verfahren zeichnet sich dadurch aus, dass mittels eines äußerst kompakten und robusten photoakustischen Gassensors, auf Basis von MEMS-Technologie, zuverlässig die Präsenz und/oder Konzentration der Aerosolpartikeln detektiert werden kann. Im Gegensatz zu vielen Ansätzen im Stand der Technik ist vorteilhaft, dass hierzu keine aufwändige Probenmanipulation notwendig ist. Stattdessen kann durch eine Abstimmung der Wellenlänge der emittierten Strahlung auf das Absorptionsverhalten der Aerosolpartikeln zuverlässig die Präsenz und/oder Konzentration der Aerosolpartikel, insbesondere im Hinblick auf biologische Strukturen wie Viren oder Bakterien, festgestellt werden.

Das Verfahren zeichnet sich somit durch eine einfache und schnelle Erkennung der Aerosolpartikel in der Luft durch einen photoakustischen Effekt aus. Durch die kostengünstige Implementation unter Nutzung eines kompakten MEMS -basierten Sensors ergibt sich ein überaus breites Spektrum an Anwendungsmöglichkeiten.

Das Verfahren kann beispielsweise in öffentlichen Räumen, öffentlichen Verkehrsmitteln, aber auch im privaten Umfeld oder Lüftungsanlagen verwandt werden, um zuverlässig die Umgebungsluft im Hinblick auf die Präsenz und/oder Konzentration von Aerosolpartikeln, insbesondere Viren oder Bakterien zu überwachen. Im Falle der Überschreitung zuvor definierter Grenzwerte kann eine Warnung ausgegeben und/oder Maßnahmen zur Vermeidung einer Ausbreitung von potentiell gefährlichen Aerosolpartikel ergriffen werden.

Besondere Bedeutung erlangt das Verfahren für den medizinischen Bereich, beispielsweise als Frühwarnsystem in ärztlichen Wartezimmern oder in den verschiedensten Bereichen eines Krankenhauses. Die Einfachheit des Verfahrens und Möglichkeit einer kostengünstigen Umsetzung erlaubt hierbei insbesondere eine großzügige Abdeckung zur Überwachung der Umweltluft im Hinblick auf die Ausbreitung pathogener Partikel.

In besonders bevorzugten Ausführungsformen des Verfahrens handelt es sich bei den Bioaerosolpartikeln bevorzugt um Pollen, Sporen, Bakterien und Viren. Auch können mit dem Verfahren auch Rußpartikel oder Staubpartikel detektiert werden. Besondere Bedeutung erlangt das Verfahren jedoch durch die von den Erfindern erkannte Möglichkeit, dass eine PAS auch zur spezifischen Detektion von komplexen biologischen Strukturen wie Bakterien, Viren, Pollen oder auch Sporen eingesetzt werden kann.

Ein photoakustisches Spektrometer für die Analyse von Gas ist dem Fachmann dabei in seinen Grundzügen bzw. wesentlichen Komponenten bekannt. Ein modulierbarer Emitter erzeugt elektromagnetische Strahlung, insbesondere im infraroten Wellenlängenbereich und ist dabei bevorzugt so angeordnet und konfiguriert, dass die von dem Emitter emittierte Strahlung im Wesentlichen oder zumindest teilweise auf das zu analysierende Gas in der Messzelle trifft. Erfolgt die modulierte Bestrahlung mit einer Infrarotwellenlänge, welche dem Absorptionsspektrum eines in dem Gasgemisch befindlichen Moleküls einer Gaskomponente entspricht, findet eine modulierte Absorption statt, die zu Erwärmungs- und Abkühlungsprozessen führt, deren Zeitskalen die Modulationsfrequenz der Strahlung widerspiegeln. Gemäß dem photoakustischen Effekt führen die Erwärmungs- und Abkühlungsprozesse zu Expansionen und Kontraktion der Gaskomponente wodurch die Gaskomponente zur Ausbildung von Schalldruckwellen mit im Wesentlichen der Modulationsfrequenz angeregt werden kann. Diese lassen sich durch den Schalldetektor messen. Die Leistung der Schallwellen ist dabei vorzugsweise direkt proportional zur Konzentration der absorbierenden Gaskomponente. Überraschend wurde festgestellt, dass sich der photoakustische Effekt in ähnlicher Weise auf komplexe und im Vergleich zu Gaskomponenten makroskopische biologische Strukturen erweitern lässt und somit zur Detektion von Bioaerosolen geeignet ist.

In einer bevorzugten Ausführungsform erfolgt eine selektive Anregung und Detektion der Aerosolpartikel durch eine Abstimmung einer oder mehrerer Wellenlänge der emittierten Strahlung auf das Absorptionsverhalten der Bioaerosolpartikel, wie beispielsweise Pollen, Sporen, Bakterien oder Viren.

Zu diesem Zweck kann es bevorzugt sein, dass Referenzdaten über das wellenlängenabhängige photoakustische Absorptionsverhalten der Aerosolpartikel aufgezeichnet werden, um die wellenlängenabhängige Anregung zu optimieren.

Vorteilhaft weisen Bioaerosole, wie beispielsweise Pollen, Sporen, Bakterien und Viren zu diesem Zwecke einen charakteristischen photoakustischen Fingerabdruck auf. So treten für die untersuchten biologischen Strukturen wellenlängenabhängig distinkte photoakustische Resonanzen auf.

Hierbei zeigt sich, dass häufig für Wellenlängen im nahen Infrarot-Bereich für Bioaerosole, wie Bakterien, Viren oder Pollen, an einer oder mehrerer Wellenlängen ein charakteristisches photoakustisches Signal verzeichnet werden kann.

Ebenso kann jedoch auch im UV-Bereich bestimmte Bestandteile der biologischen Strukturen selektive angeregt werden. Carotinoide im der Pollenhülle absorbieren im blauen Bereich (Wellenlängen über 500 nm) des sichtbaren Lichts. Proteine, Biomakromoleküle und Nucleinsäuren besitzen Chromophore, welche in der Regel bei Wellenlängen unter 300nm (UV-Bereich) absorbieren. Proteine in der Virushülle absorbieren somit im UV-Bereich. Wasser und organische Strukturen absorbieren auch das energiearme Infrarot-Licht besonders gut. Versuchsreihen zeigen jedoch, dass bei der Untersuchung von Aerosolen, insbesondere Bioaerosolen auf Grund des hohen Wasseranteiles bevorzugt Wellenlängen über 170 nm zum Einsatz kommen.

In einer besonders bevorzugten Ausführungsform des Verfahrens handelt es sich bei den Bioaerosolpartikeln bevorzugt um Pollen, Sporen, Bakterien oder Viren, wobei die eine oder mehrere Wellenlängen des Emitters zur selektiven Anregung bevorzugt aus einem Bereich zwischen 170 nm und 10 µm, bevorzugt zwischen 200 nm und 1000 nm gewählt werden. Bevorzugte anregende Wellenlängen liegen in dem vorgenannten Bereich, wobei auch Zwischenbereiche bevorzugt seien können wie beispielsweise 200 nm bis 300 nm, 300 nm bis 400 nm, 400 nm bis 500 nm, 600 nm bis 700 nm, 700 nm bis 800 nm, 800 nm bis 900 nm, 900 nm bis 1000 nm, 1000 nm bis 1500 nm, 1500 nm bis 2000 nm, 2500 nm bis 3000 nm, 3000 nm bis 4000 nm, 4000 nm bis 5000 nm oder auch 5000 nm bis 10 000 nm. Ein Fachmann erkennt, dass die vorgenannten Bereichsgrenzen auch kombiniert werden können, um weitere bevorzugte Bereiche zu erhalten, wie beispielsweise 200 nm bis 1500 nm, 500 nm bis 900 nm oder 300 nm bis 2000 nm.

Die Auswahl der einen oder mehreren Wellenlängen für einen spezifischen Biopartikel, beispielsweise einen Virus, Pollen und/oder Bakterium lässt sich wie obig beschrieben bevorzugt anhand von Versuchsreihen ermitteln.

Besonders gute Ergebnisse können bei der Anregung mit zwei, drei, vier, fünf oder mehr verschiedenen Wellenlängen, insbesondere aus dem Bereich zwischen 170 nm und 10 µm, bevorzugt 200 nm und 1000 nm erfolgen. Hierbei kann es bevorzugt sein, dass die selektive Anregung mit den zwei, drei, vier, fünf oder mehr verschiedenen Wellenlängen zu zwei, drei, vier, fünf oder mehr verschiedenen Zeitpunkten erfolgt und die Feststellung der Präsenz und/oder Konzentration der Aerosolpartikeln in der Umgebungsluft anhand der gemessenen Schalldruckwellen an den zwei, drei, vier, fünf oder mehr verschiedenen Wellenlängen erfolgt.

Erfindungsgemäß wurde festgestellt, dass aufgrund der komplexen Zusammensetzung der biologischen Strukturen, beispielsweise im Hinblick deren Proteinzusammensetzung, verschiedenen biologischen Strukturen in hohem Maße aussagekräftige photoakustische Fingerabdrücke zugeordnet werden können.

Zwar können die biologischen Aerosolpartikel, wie Viren, Bakterien oder Pollen, grundsätzlich ähnliche Proteine umfassen, sodass photoakustische Absorptionen in ähnlichen Wellenlängenbereichen auftreten. Der unterschiedliche Anteil der Proteine führt jedoch zu besonders ausgeprägten PAS Signalen an den verschiedenen Wellenlängen. Informationen über die wellenlängenabhängige Position der PAS Signale sowie deren jeweiligen Amplitude, erlauben somit eine besonders präzise Feststellung der Präsenz oder Konzentration spezifischer Aerosolpartikel, analog zu einem spektralen Fingerabdruck.

Der Begriff PAS Signal oder photoakustisches Signal meint bevorzugt die Messergebnisse des Sensors über die im Analysevolumen erzeugten Schalldruckwellen, insbesondere Schalldruckwellen mit einer Frequenz, welche im Wesentlichen der Modulationsfrequenz des anregenden Emitters entspricht.

In bevorzugten Ausführungsformen werden die Referenzdaten anhand von Kalibrationsmessungen erhalten, bei denen die Konzentration der zu detektierenden Aerosolpartikel in der Umgebungsluft bekannt ist und kontrolliert wird. Hierzu kann der photoakustische Gassensor beispielsweise in eine Kalibrationskammer gebracht werden, in welcher unter Laborbedingung eine definierte Konzentration von Aerosolpartikel vorliegen.

Zum Erhalt der Referenzdaten erfolgt bevorzugt eine wellenlängenabhängige Detektion der photoakustischen Signale über einen definierten Wellenlängenbereich, beispielsweise von 170 nm bis 10 µm, bevorzugt 170 nm bis 1000 nm umfasst. Anhand der detektierten Peaks der photoakustischen Signale erfolgt eine Festlegung der eine oder mehreren Wellenlängen zur selektiven Anregungen sowie Grenzwerte zu den korrespondierenden photoakustischen Signalen, welche eine erhöhte Konzentration der Aerosolpartikel in der Umgebungsluft anzeigen.

Vorteilhaft kann durch das vorgeschlagene Verfahren eine Vielzahl auch komplexer Aerosolpartikel in der Umgebungsluft detektiert werden.

Im Sinne der Erfindung bezieht sich der Begriff Aerosol im Allgemeinen auf eine Ansammlung von flüssigen oder festen Partikeln (oder Teilchen), welche in einem gasförmigen Medium lange genug suspendiert sind, um beobachtet und gemessen zu werden. Die Partikel werden auch als Aerosolpartikel bzw. Schwebeteilchen bezeichnet. Die Größe von Aerosolpartikel reicht typischerweise von etwa 0,001 µm bis etwa 100 µm (vgl. Kulkarni et al., Aerosol Measurement, 3rd ed., John Wiley & Sons, Inc., 2011).

Der Begriff Umgebungsluft bezieht sich im Allgemeinen auf ein Gas (oder gasförmiges Fluid, oder Gasphasenfluid). Das Gas kann flüssige Tröpfchen oder Dampf enthalten oder auch nicht und kann Aerosolpartikel enthalten oder auch nicht. Ein Aerosol kann daher auch so betrachtet werden, dass es Partikel und ein Gas enthält, das die Partikel mitreißt oder trägt.

Wie hier verwendet, bezieht sich der Begriff Bioaerosol im Allgemeinen auf ein Aerosol, in dem ein oder mehrere Biopartikel suspendiert oder mitgeführt werden. Der Begriff Biopartikel bezieht sich im Allgemeinen auf ein biologisches Material oder die Kombination aus einem biologischen Material und einem nicht-biologischen Partikel, auf dem das biologische Material getragen wird. Das heißt, ein biologisches Material kann selbst ein Partikel sein, der frei in einem Aerosol suspendiert ist, oder es kann auf einem nicht-biologischen Partikel getragen werden, so dass das biologische Material und der nicht-biologische Partikel zusammen im Aerosol suspendiert sind.

Das biologische Material kann auf dem nichtbiologischen Partikel durch einen beliebigen Mechanismus getragen werden, wie z.B. Einschluss, Einbettung, Adhäsion, Adsorption, Anziehungskraft, Affinität usw. Beispiele für biologisches Material sind unter anderem Sporen (z.B. Pilzsporen, Bakteriensporen usw.), Pilze, Schimmelpilze, Bakterien, Viren, biologische Zellen oder intrazelluläre Komponenten, biologisch abgeleitete Partikel (z.B. Hautzellen, Detritus usw.) etc.

Bioaerosole können pathogene oder nicht-pathogene, lebende oder tote Bakterien und Pilze, Viren, hochmolekulare Allergene, bakterielle Endotoxinen, Mykotoxinen, Peptidglykanen, Beta(1-3)-Glucanen, Pollen, Pflanzenfasern etc. umfassen. Die Exposition gegenüber Bioaerosolen ist mit einer Reihe von Krankheiten wie Infektionskrankheiten und Atemwegserkrankungen verbunden. Andere Krankheiten und Zustände wurden mit einer Exposition mit Bioaerosolen in Verbindung gebracht, wie beispielsweise Krebserkrankungen, (Bioaerosol Health Effects and Exposure Assessment: Progress and Prospects, J. Douwes, P. Thorne, N. Pearce und D. Heederik, Institute for Risk Assessment Sciences, Division of Environmental and Occupational Health, Universität Utrecht, Niederlande; Centre for Public Health Research, Massey University Wellington Campus, Wellington, Neuseeland; University of lowa College of Public Health, Department of Occupational and Environmental Health, IA, USA).

Die biologischen Mikroorganismen bzw. Partikel können in der Umgebungsluft auf verschiedene Weise gelangen: Zum Beispiel als isolierte Partikel ohne wesentlichen Anteil von Begleitsubstanzen; an festen Teilchen haftend, z.B. Hautschuppen, Pflanzenteilen, Bodenpartikel oder auch in Tröpfchen.

Insbesondere die Tröpfchenübertragung stellt einen wesentlichen Infektionsweg zur Übertragung aerogener Infektionskrankheiten dar. Beim Ausatmen, Sprechen, Erbrechen sowie beim Niesen und Husten werden Speichel und andere flüssige Absonderungen der Atemwege wie Nasensekret und Sputum durch Vernebelung als Tröpfchen an die Umgebung abgegeben.

Der Begriff Bioaerosol soll sämtliche vorgenannte Schwebemechanismen umfassen. D.h. unter dem Begriff Bioaerosol sollen insbesondere sowohl einzelne Biopartikel, wie beispielweise frei schwebende Pilzsporen, Bakterien und Hefen welche auf anderen Partikeln haften und mitschweben (Hautschuppen, Staub, Pflanzenteile, Haare, Federn, Fasern von Bekleidung) oder Tröpfchen in denen sich Bakterien oder Viren befinden verstanden werden.

In einer bevorzugten Ausführungsform der Erfindung sind die zu detektierenden Aerosolpartikeln Viren.

Der Begriff "Virus", wie er hier verwendet wird, bezieht sich bevorzugt auf einen kleinen infektiösen Erreger, der sich nur in den lebenden Zellen von Organismen vermehren kann und kann Virionen wie auch Viruspartikel umfassen. Bevorzugt können Viren einen Durchmesser von 20 - 330 nm aufweisen.

Nicht einschränkende Beispiele von Virusfamilien sind Adenoviridae, Arenaviridae, Bunyaviridae, caliciviridae, circoviridae, coronaviridae, deltavirus, Siphoviridae, filoviridae, flaviviridae, hepadnaviridae, hepeviridae, herpesviridae, orthomyxoviridae, paramyxoviridae, picomaviridae, poxyviridae (Pockenviren), reoviridae, retroviridae und rhabdoviridae.

Besonders bevorzugt handelt es sich um Viren, welche über den Luftweg übertragen werden und für verschiedene Krankheiten bei Menschen, Tieren und Pflanzen verantwortlich sind, wie zum Beispiel Windpocken (durch das Varizella-Zoster-Virus, VZV), Erkältung (durch das Coronavirus), Lungenerkrankungen, insbesondere COVID 19 durch das SARS-CoV-2 Virus, Grippe bei Menschen und Tieren (Influenzaviren), Masern (Masernviren), Röteln Rinderpest (durch das Morbillivirus) oder Atemwegserkrankungen bei Rindern (durch das Bovine Respiratory Syncytial Virus, BRSV) oder auch Pflanzenviren, die aus dem Boden aerosoliert werden.

In einer bevorzugten Ausführungsform der Erfindung sind die zu detektierenden Aerosolpartikeln Bakterien. Die Größe, Form oder Art von Bakterien, welche mittels des vorgeschlagenen Verfahrens detektierten werden kann, ist sehr unterschiedlich. Ein Durchmesser der Bakterien kann beispielsweise zwischen etwa 0,1 und 700 µm liegen, bevorzugt zwischen etwa 0,6 bis 1,0 µm.

Bakterien kommen in verschiedenen äußeren Formen vor: kugelförmig, sogenannte Kokken (Micrococcus), zylinderförmig, sogenannte Stäbchen (Bacillus, Escherichia) mit mehr oder weniger abgerundeten Enden, wendelförmig (Spirillen, Spirochäten), mit Stielen (Caulobacter), mit Anhängen (Hyphomicrobium), mehrzellige Trichome bildend (Caryophanon, Oscillatoria), lange, verzweigte Fäden, sogenannte Hyphen, bildend, die sich verzweigen und eine Mycel genannte Fadenmasse bilden (Streptomyzeten), sowie Gebilde mit mehreren unregelmäßig angeordneten Zellen (Pleurocapsa). Bakterien können auch in Aggregaten vorkommen: Kugelketten (Streptococcus), flächige Anordnung kugelförmiger Zellen (Merismopedia), regelmäßige dreidimensionale Anordnung von Kugeln (Sarcina), Stäbchenketten (Streptobacillus), in Röhren eingeschlossene Stäbchenketten (Leptothrix).

Besonders bevorzugt handelt es sich um Bakterien, welchen über den Luftweg übertragen werden und für verschiedene Krankheiten bei Menschen, Tieren und Pflanzen verantwortlich sind, wie zum Beispiel Tuberkulose (durch das Mycobacterium tuberculosis), Diphterie (Corynebacterium diphtheriae), Scharlach, Meningitis (durch Streptokokken) oder auch Lungenentzündung (durch Pneumokokken, eine Unterart der Streptokokken).

In einer bevorzugten Ausführungsform der Erfindung sind die zu detektierenden Aerosolpartikeln Pilze oder Pilzsporen. Infektionskrankheiten, welche von Pilzen ausgelöst werden, werden auch als Mykosen oder Pilzerkrankungen benannt. Hierzu gehören sowohl oberflächliche Mykosen, beispielsweise Mykosen der Haut oder Schleimhäute sowie systemischen Mykosen, bei denen die Pilzerreger zumeist über die Lunge in den Blutkreislauf gelangen.

Mittels des vorgeschlagenen Verfahrens können vorteilhaft Pilze oder Pilzsporen erfasst werden, welche über die Umgebungsluft übertragen werden, wie Schimmelpilzsporen. Insbesondere bei Personen mit geschwächtem Immunsystem aufgrund einer Vorerkrankung (u.a. HIV, Diabetes) oder auch aufgrund der Einnahme von immunsupprimierenden Medikamenten (im Falle von Autoimmunerkrankungen) stellen Pilzinfektionen eine hohe Gefahr dar. Eine kontinuierliche Überwachung der Präsenz oder Konzentration von Pilzen bzw. Pilzsporen kann daher im medizinischen Bereich beispielsweise im Krankenhaus eingesetzt werden, um Folgeerkrankungen zu verhindern.

In einer bevorzugten Ausführungsform der Erfindung sind die zu detektierenden Aerosolpartikeln Pollen. Pollen oder Blütenstaub ist zumeist eine mehlartige Masse, die in den Staubblättern der Samenpflanzen gebildet wird und besteht aus Pollenkörnern. Pollenkörner sind nach Größe, Form und Oberflächenstruktur sehr vielgestaltig und lassen sich vielfach aufgrund dieser Merkmale den jeweiligen Arten oder zumindest Gattungen zuordnen. Die meisten Pollenkörner sind zwischen 10 und 100 Mikrometer groß.

Häufig enthalten Pollen allergene Proteine, welche die Hauptursache für allergische Erkrankungen wie Heuschnupfen oder Asthma in gemäßigten Klimata ist (Marsh, 1975). Als aerogene Partikel können Pollen über viele Kilometer sowie Stunden und Tage in der Luft sich ausbreiten. Eine routinemäßige Überwachung der Konzentration von Pollen in der Umgebungsluft mittels des vorgeschlagenen Verfahrens erlaubt mithin ein frühzeitiges Warnsystem zu etablieren, welches potentiell gefährdete Personen vor allergischen Überreaktion schützen kann.

Die Erfinder haben erkannt, dass Bioaerosole (u.a. Bakterien, Viren, Sporen oder Pollen) zuverlässig mit einem photoakustischen Gassensor umfassend einen modulierbaren Emitter und einen Schalldruckdetektor detektiert werden können

Ein modulierbarer Emitter bezeichnet bevorzugt eine Vorrichtung, die elektromagnetische Strahlung aussendet.

Diese Strahlung hat bevorzugt einen Wellenlängenbereich im Infrarot (IR) Bereich, insbesondere zwischen ca. 700 Nanometer (nm) und 10 µm, im ultravioletten Bereich, insbesondere zwischen 170 nm und 380 nm oder auch im sichtbaren Bereich (VIS), insbesondere zwischen 380 nm und 700 nm.

Das Spektrum ist insbesondere so gewählt, dass es dem bevorzugten Anwendungsgebiet der photoakustischen Spektroskopie entspricht.

Dabei ist insbesondere die Schwingungsanregung der zu spektroskopierenden und/oder zu detektierenden Aerosolpartikel bevorzugt, welche je nach Bestandteilen der Bioaerosole einem bevorzugten spektralen Bereich entsprechen.

Carotinoide im der Pollenhülle absorbieren bevorzugt im sichtbaren blauen Bereich (Wellenlängen über 500 nm). Proteine, Biomakromoleküle und Nucleinsäuren besitzen häufig Chromophore, welche nur bei Wellenlängen unter 300 nm (UV-Bereich) absorbieren. Proteine in der Virushülle absorbieren häufig ebenfalls im UV-Bereich. Organische Moleküle, welche in Bakterien und Viren auftreten, absorbieren zudem das energiearme Infrarot-Licht (insbesondere Nah-Infrarotlicht) besonders gut.

In einer bevorzugten Ausführungsform ist der Emitter ein Infrarot-Emitter. Besonders bevorzugte Wellenlängenbereiche der Infrarotstrahlung sind 700 nm bis 10 µm, bevorzugt 700 nm bis 3 µm oder auch 700 nm bis 1 µm.

Zur Erzeugung der Infrarotstrahlung ist bevorzugt die Bereitstellung thermischer Energie in Form eines Heizelements vorgesehen. Besonders bevorzugt ist ein (Mikro-) Heizelement. Unter einem Mikro-Heizelement wird bevorzugt ein Heizelement mit Abmessungen der Größenordnung Mikrometer (µm) verstanden. Dabei umfasst das Heizelement eine erhitzbare Schicht aus einem leitfähigen Material, welches bei Durchfluss eines elektrischen Stroms joulesche Wärme produziert. Die produzierte Wärme zeigt bevorzugt eine Abhängigkeit vom ohmschen Widerstand des Elements und vom Quadrat der Stromstärke bzw. vom Quadrat der angelegten Spannung und dem inversen ohmschen Widerstand, je nachdem, ob eine Strom- oder eine Spannungsquelle verwendet wird. Eine thermische Quelle infraroter Strahlung hat für die PAS vorteilhafte Eigenschaften, wie z. B. eine breitbandige Emission, durch die eine Vielzahl verschiedenster Gasatome bzw. -moleküle mit nur einer Lichtquelle angeregt werden können. Gleichzeitig ist ein thermischer IR-Emitter besonders kostengünstig, einfach herzustellen und langlebig.

In einem Gleichgewichtszustand ist die produzierte Wärme gleich zu den Wärmeverlusten durch Wärmeleitung, Konvektion und Wärmestrahlung (synonym: thermische Strahlung, Infrarotstrahlung), welche an den äußeren Grenzflächen der stromdurchflossenen erhitzbaren Schicht abgegeben wird. Wie dem Fachmann bekannt ist, verursacht die produzierte Wärme unter anderem thermische Strahlung, insbesondere durch thermische Bewegung von Teilchen, welche z. B. eine Beschleunigung von Ladungsträgern und/oder oszillierende Dipolmomente zur Folge hat. Somit kann durch eine stromdurchflossene erhitzbare Schicht gezielt Infrarotstrahlung erzeugt werden. Die erhitzbare Schicht ist bevorzugt aus Metall, beispielsweise aus Wolfram oder aus Platin. Durch Anlegen einer geeigneten Spannung und den daraus resultierenden Stromfluss wird joulesche Wärme und somit letztendlich Infrarotstrahlung erzeugt.

Das Strahlungsspektrum eines erhitzen Körpers lässt sich dabei bevorzugt angenähert durch das Planck'sche Strahlungsgesetz beschreiben, wobei dem Fachmann die Unterschiede einer realen erhitzbaren Schicht zu einem schwarzen Körper bekannt sind, beispielsweise der Emissionsgrad oder die reale Abweichung von einem thermischen Gleichgewicht des Körpers. Trotz dieser Abweichungen werden das erzeugte Spektrum und dessen Intensität im Wesentlichen von der Temperatur und der abstrahlenden Fläche gemäß dem Planck'schen Strahlungsgesetz beschrieben.

Begriffe wie im Wesentlichen, ungefähr, etwa, ca. etc. beschreiben bevorzugt einen Toleranzbereich von weniger als ± 20%, bevorzugt weniger als ± 10 %, noch stärker bevorzugt weniger als ± 5% und insbesondere weniger als ± 1%. Angaben von im Wesentlichen, ungefähr, etwa, ca. etc. offenbaren und umfassen stets auch den exakten genannten Wert.

Somit kann ein Fachmann durch gezieltes Design eines (Mikro-) Heizelements ein bevorzugtes Spektrum mit einer bevorzugten Intensitätsverteilung erzielen. Hierzu sind neben dem Material und der geometrischen Ausgestaltung des Heizelements bevorzugt die zur Verfügung gestellte elektrische Energie, sowie die Größe der Wärmeverluste des Heizelements neben der Wärmestrahlung maßgeblich. Die Größe dieser Wärmeverluste wird beispielsweise bestimmt durch die Wärmeleitfähigkeit zwischen dem Heizelement und den angrenzenden Materialien und/oder Fluiden sowie deren Wärmekapazität und der Größe der Grenzfläche(n).

Ein IR- Emitter in Form eines Heizelements ist besonders kostengünstig und robust, gleichzeitig lässt sich aufgrund der spektralen Breite der Emission eine Vielzahl Aerosolpartikeln bei einer PSA detektieren. Durch einen vorzugsweise durchstimmbaren Bandpass-Filter lassen sich bevorzugt bei Bedarf schmalere Spektren aus dem breiten Emissionsspektrum selektieren.

Die Infrarotstrahlung eines Infrarot-Emitters kann vorzugsweise ebenso durch eine im gewünschten infraroten Spektralbereich emittierende Leuchtdiode (LED) und/oder einen Laser erzeugt werden.

In einer bevorzugten Ausführungsform ist der Emitter eine VIS- und/oder UV-Emitter. Besonders bevorzugte Wellenlängenbereich der UV-Strahlung sind 170 nm bis 380 nm, während durch den VIS-Emitter (*visible* Emitter im sichtbaren Bereich) ein bevorzugter Wellenlängenbereich zwischen 380 nm und 700 nm abgedeckt werden kann.

Für einen VIS- oder UV-Emitter können besonders bevorzugt emittierende Leuchtdiode (LED) und/oder einen Laser zum Einsatz.

LEDs sind heutzutage für verschiedenste Wellenlängenbereiche kostengünstig in kompakter Bauweise erhältlich. Laser weisen vorzugsweise ein schmales Emissionsspektrum auf, so dass bevorzugt nur genau auf dieses Spektrum passende Absorptionslinien der Bioaerosolpartikel angeregt und somit detektiert werden können.

Die Emission des Emitters erfolgt bevorzugt als Strahl, welcher in einer bevorzugten Richtung in Form einer Grade orientiert ist. Der Begriff Strahl bezeichnet bevorzugt den gebündelten Teil der Strahlung entlang der bevorzugten Strahlrichtung des Emitters, wobei insbesondere die Bereiche der größten Intensität entlang dieser Richtung den Strahl definieren. Intensität ist bevorzugt definiert als Flächenleistungsdichte und hat bevorzugt die Einheit Watt pro Quadratmeter oder abgekürzt W/m².

Es können zusätzliche Komponenten wie z. B. Linsen im Emitter integriert oder extern angebracht sein, die für eine Bündelung bzw. Kollimation des Strahls sorgen. Ein Fachmann weiß, wie er durch das Design des Emitters sowie durch Verwendung weiterer Komponenten das Emissionsprofil der Strahlungsquelle so formt, dass ein gewünschtes Strahlprofil sowie eine gewünschte Strahlrichtung resultieren. Dabei kann der modulierbare Emitter bevorzugt ohne zusätzliche Linsen auskommen, oder als ein System umfassend Strahlungsquelle und mindestens einer Linse zur Kollimation des Strahls vorliegen.

Der Emitter ist modulierbar, das bedeutet, dass die Intensität der emittierten Strahlung, bevorzugt die Intensität des Strahls im zeitlichen Verlauf kontrollierbar geändert werden kann. Die Modulation soll bevorzugt eine zeitliche Änderung der Intensität als messbare Größe hervorrufen.

Das bedeutet z. B., dass die Intensität im zeitlichen Verlauf zwischen der innerhalb des Messzeitraums gemessenen schwächsten Intensität und der innerhalb desselben Zeitraums gemessenen stärksten Intensität ein Unterschied besteht, der größer ist als die Sensibilität eines für das Strahlungsspektrum und die Anwendung typischerweise verwendeten Geräts zur Messung oder Bestimmung der Intensität. Bevorzugt ist der Unterschied deutlich größer als ein Faktor 2, mehr bevorzugt 4, 6 oder 8 zwischen der stärksten und der schwächsten einstellbaren Intensität. Besonders bevorzugt erfolgt die Modulation der Intensität des modulierten Strahles für eine oder mehrere vorbestimmte Wellenlängen mit denen eine selektive Anregung der Bioaerosolpartikel, wie Bakterien, Viren, Sporen oder Pollen, erfolgt. Vorzugsweise kann beispielsweise bei einem thermischen Infrarot-Emitter oder einer LED eine direkte Modulation durch Variation der Stromzufuhr vorgenommen werden. Diese ist ebenfalls besonders einfach und kostengünstig zu realisieren.

Eine Modulation des Emitters kann vorzugsweise ebenso durch eine externe Modulation erfolgen, z. B. durch die Verwendung eines sich drehend Chopperrades und/oder eines elektrooptischen Modulators.

In einer bevorzugten Ausführungsform erlaubt der modulierbare Emitter eine wellenlängenselektive Abstrahlung und/oder im Strahlengang zwischen dem modulierbaren Emitter und der Detektionskammer liegt ein wellenlängenselektiver Filter, beispielsweise ein Fabry-Perot-Filter, vor.

Der wellenlängenselektive Filter ist bevorzugt durchstimmbar. So kann der photoakustische Gassensor zur Feststellung der Präsenz und/oder der Konzentration verschiedener Bioaerosolpartikel verwendet werden, welche bei einer oder mehrerer Wellenlängen zur PAS angeregt werden können. Vorteilhaft kann hierbei auch eine Anregung mittels mehrerer Wellenlängen zeitlich sukzessive erfolgen, sodass die Anregung auf das wellenlängenabhängige Absorptionsverhalten der zu detektierende Aerosolpartikel abstimmbar ist.

Ein wellenlängensensitiver Infrarotemitter kann z. B. ein durchstimmbarer Laser sein und/oder mehrere Laser verschiedener Wellenlängen umfassen.

Bei Verwendung eines durchstimmbaren Filters kann insbesondere ein Emitter mit einem breiten Spektrum zum Einsatz kommen, z. B. eine LED und/oder ein thermischer Emitter im Falle eines IR-Emitters.

In einer bevorzugten Ausführungsform ist der modulierbare Emitter ein MEMS-Emitter.

Die MEMS-Technologie bezeichnet insbesondere eine Technologie für die Herstellung kompakter, mechanisch-elektronischer Vorrichtungen mittels der Mikrosystemtechnik. Die so herstellbaren Mikrosysteme (engl. *microelectromechanical system,* kurz MEMS) sind sehr kompakt (Mikrometerbereich) bei gleichzeitig hervorragender Funktionalität und immer geringeren Herstellungskosten.

Sowohl der Emitter selbst als auch mikromechanische Strukturen zu dessen Modulation können hierbei durch MEMS-Technologie bereitgestellt werden.

Beispielsweise kann die Modulation der Intensität des Emitters mittels MEMS-Aktuatoren erfolgen, welche eine Relativbewegung zwischen einer Blendenstruktur und einer Strahlungsquelle steuern (vgl. u.a. EP 3623779 A1 oder EP 36323780 A1).

Durch die Verwendung von MEMS-Aktuatoren, beispielsweise einem elektrostatischen Aktuator, einem piezoelektrischen Aktuator, einem elektromagnetischen Aktuator und/oder einem thermischen Aktuator kann auf besonders schnelle und einfache Weise eine Modulation der Intensität der emittierten Infrarotstrahlung erreicht werden. Insbesondere können Modulationsfrequenzen von deutlich über 100 Hz bis hin zu 100 kHz erreicht werden. Derartige Modulationsfrequenzen sind insbesondere für die photoakustische Spektroskopie vorteilhaft zur Erhöhung des Signal-Rausch-Verhältnisses. So kann die Modulationsfrequenz des Emitters in einen Bereich geführt werden, der weiter entfernt vom Eigenrauschen der Detektionskomponenten eines Schalldetektors, wie beispielsweise eines Mikrofons ist. Bei Mikrofonen ist das Eigenrauschen insbesondere in diesem Bereich von einigen Hz bis ca. 100 Hz hoch.

Das Analysevolumen stellt ein offenes System dar, welches eine oder mehrere Öffnungen aufweist, sodass die Umgebungsluft umfassend Aerosolpartikel in das Analysevolumen einströmen oder diffundieren kann.

In einer bevorzugten Ausführungsform ist das Analysevolumen nach außen zumindest bereichsweise abgeschlossenes oder abschließbares Volumen (bzw. Kammer), in der sich die Umgebungsluft befindet bzw. eingeleitet werden kann, z. B. durch eine oder mehrere Öffnung in Form, welches auch verschließbar durch einen Verschlusses, Ventils und/oder durch eine Zuleitung gestaltet sein kann.

Das Analysevolumen ist vorzugsweise somit zumindest teilweise geöffnet sein. Dadurch kann insbesondere eine das Spektroskop umgebende Gasatmosphäre (Luft), welche gegenüber dem Analysevolumen zumindest teilweise geöffnet ist, vermessen und auf ihren Anteil bzw. Konzentration an Aerosolpartikeln überprüft werden.

Vorteilhaft ist in diesem Fall, dass das Analysevolumen wohl definiert ist, so dass der modulierbare Emitter, das Analysevolumen und der Schalldetektor derart angeordnet vorliegen, dass die von dem Emitter modulierbar emittierbare Strahlung Aerosolpartikel im Analysevolumen zur Ausbildung von Schalldruckwellen anregen kann, welche mit Hilfe des Schalldruckdetektors messbar sind.

Das Analysevolumen liegt bevorzugt im Strahlengang des Emitters vor. Das bedeutet bevorzugt, dass die Intensität des Strahls im Wesentlichen oder zumindest teilweise auf die dem Emitter zugewandte Seite des Analysevolumens trifft. Teilweise bedeutet bevorzugt zu mindestens 40 %, bevorzugt mindestens 50%, 60%, 70%, 80% oder mehr.

In einer bevorzugten Ausführungsform kann der Emitter von außen auf einen Vorzugsbereich des Analysevolumens ausgerichtet werden. Muss dabei durch eine Außenwand des Volumens hindurch gestrahlt werden, um die Aerosolpartikel im Inneren anzuregen, ist die Außenwand bevorzugt zumindest in diesem Bereich für die Strahlung (z.B. IR, UV und/oder VIS) im Wesentlichen transparent. Der Emitter kann aber auch im Inneren des Analysevolumen angeordnet vorliegen.

Bei einem Analysevolumen, welches zumindest teilweise geöffnet ist und somit vorzugsweise einen permanenten Gasaustausch mit einer Umgebung zulässt, findet eine Befüllung des Analysevolumens durch Wechselwirkung mit einer Gasatmosphäre der Umgebung statt.

Ein Analysevolumen kann vorzugsweise eine Probekammer und eine Referenzkammer umfassen, welche durch einen Verbindungskanal verbunden bzw. verbindbar sind.

Im Falle einer Ausführungsform eines Analysevolumens, welche eine Probekammer und eine Referenzkammer aufweist, kann es bevorzugt sein, in jede Kammer mindestens einen Schalldetektor einzubringen, um in jeder Kammer separat zu messen und somit Störquellen, z. B. externe Schalldruckwellen, welche nicht von der in der Probekammer absorbierten Strahlung herrühren, vorzugsweise nach der Messung herausrechnen zu können.

Ebenso kann es bevorzugt sein, dass der Emitter die Probekammer und nicht die Referenzkammer bestrahlt und wobei zwischen der Probekammer und Referenzkammer ein Verbindungskanal vorliegt, in welchem sich ein Schalldetektor befindet. Diese Ausführungsform zeichnet sich durch eine besonders präzise photoakustische Spektroskopie aus, da beispielsweise Schall von unerwünschten Schallquellen bei der Messung und/oder der Auswertung der Messung herausgerechnet bzw. nicht mitgemessen wird. Vorzugsweise können Probenvolumen und ein Referenzvolumen im Wesentlichen gleiche Abmessungen aufweisen, um eine genaue differentielle Messmethode zu realisieren.

In dem Probenvolumen und Referenzvolumen kann sich das gleiche Gas befinden. Es kann ebenso bevorzugt sein, dass im Probevolumen und im Referenzvolumen unterschiedliches Gas umfasst ist, wobei im Referenzvolumen ein Gas mit bekannten Eigenschaften vorliegt und im Probenvolumen ein zu analysierendes Gas vorzugsweise die Umgebungsluft vorliegt. Durch die Verwendung zweier Volumina und mindestens eines Schalldruckdetektors kann vorteilhafterweise eine verbesserte Eliminierung von Fehlerquellen, z. B. unerwünschten Schallwellen erfolgen, weil diese auf beide Volumina wirken und der zwischen den Volumina angeordnete Schalldruckdetektor bevorzugt im Wesentlichen nur die durch die für die photoakustische Spektroskopie relevanten von der selektiven Anregung der Aerosolpartikeln hervorgerufenen Schalldruckwellen im Probenvolumen als Differenzsignal zwischen Probenvolumen und Referenzvolumen detektiert.

In einer bevorzugten Ausführungsform ist der photoakustische Gassensor dadurch gekennzeichnet, dass nicht nur der Sensor in MEMS-Technologie ausgeführt wird, sondern die gesamte Zelle, insbesondere umfassend eine Analysevolumen, Probe- und/oder Referenzkammer stark miniaturisiert bereitgestellt werden kann. Bevorzugt beträgt die größte Ausdehnung des Gassensors weniger als 5 cm, vorzugsweise weniger als 10 mm, weniger 5 mm oder weniger.

Insbesondere ist es bevorzugt, dass der gesamte photoakustische Gassensor zur Detektion der Aerosole als MEMS realisiert wird. Dabei kann vorteilhafterweise sowohl der Sensor direkt im MEMS realisiert bzw. integriert sein. Weiterhin können zudem Kammern zur Bildung eines Analysevolumen, einer Probe- und/oder Referenzkammer vorzugsweise in einem mehrlagigen Substrat gebildet werden, wobei das Substrat insbesondere ein Grundmaterial für die Fertigung der jeweiligen Komponenten bezeichnet. Der Begriff orientiert sich insbesondere an der Halbleiterindustrie, bei dem aus dem Substrat Schaltungen gefertigt werden. Dabei kommen vorliegend aus der Halbleiterindustrie und/oder der MEMS-Fertigung bekannte Materialien und/oder Fertigungstechniken zum Einsatz, welche sich durch ihre Effizienz, Einfachheit, geringen Produktionskosten und Eignung zur Fertigung hoher Stückzahlen eignet.

Ein Substrat kann dabei durch Ätzprozesse und/oder physikalische Bearbeitungstechniken am Stück nach Wunsch bearbeitet und in der Form angepasst werden, insbesondere durch Abtragen und/oder Beseitigung von Bereichen und/oder Schichtdicken des einzelnen Substrats. Ein mehrlagiges Substrat umfasst insbesondere, mehrere, bevorzugt, 2, 3, 4, 5, 6, 7, 8, 9 oder 10 Lagen oder mehr einzelne dünne Substrate, welche einzeln bearbeitet und dann zum Gassensor, insbesondere umfassend die genannten Komponenten zusammengefügt werden kann.

Auf diese Weise können beispielsweise Probe- oder Referenzkammern gebildet werden, welche eine Höhe von 10 µm bis 2 mm, bevorzugt von 50 µm bis 1 mm, besonders bevorzugt von 100 µm bis 500 µm und/oder eine Länge oder Breite von 100 µm bis 10 mm, bevorzugt von 200 µm bis 5 mm, besonders bevorzugt von 500 µm bis 2mm aufweisen. Dies erlaubt gleichzeitig eine kompakte Bauweise und die Bildung ein ausreichendes Volumen zur photoakustischen Detektion.

Bevorzugt kann ein mehrlagiges Substrat durch Bonden von mindestens zweier Wafer gebildet werden. Durch Bonden mehrerer Lagen von einzeln vorbearbeiteten Substraten können vorteilhafterweise komplexe Komponenten von Gassensoren und insbesondere vollintegrierte, komplette Gassensoren einfach hergestellt werden.

Das Bonden von Strukturen aus vorbearbeiteten Substraten erlaubt hierbei die einfache Herstellung komplexer Strukturen, welche nur mit großem Aufwand aus einem einzelnen Wafer und/oder Substrat hergestellt werden könnten. So können Kavitäten bzw. Kammern im Gassensor mittels Bonden bereitgestellt werden, ohne dass diese aufwendig aus dem Inneren eines Rohmaterials herausgearbeitet werden müssen.

Es kann ein einfach herzustellender Gassensor mit geringer Bauhöhe und hoher Kompatibilität zu weiteren Halbleiterelemente wie z. B. elektronischen Schaltungen gefertigt werden. In bevorzugten Ausführungsformen kann zudem durch ein geeignetes Zusammenfügen der Substratschichten eine monolithische Kammer mit integriertem MEMS-Sensor realisiert werden. Monolithisch bedeutet bevorzugt, aus einem Stück bestehend, zusammenhängend und/oder fugenlos bzw. aus sehr kleinen Bauelementen untrennbar zusammengesetzt.

Der MEMS-Sensor ist ein Schalldruckdetektor, wobei der Schalldruckdetektor bevorzugt einen kapazitiv oder optisch auslesbaren, piezoelektrischen, piezoresistiven und/oder magnetischen Balken und/oder ein kapazitives, piezoelektrischen, piezoresistives und/oder optisches Mikrofon umfasst.

Die Schalldruckwellen, welche durch die PAS erzeugt werden, können vorzugsweise auf verschiedene Arten detektiert werden. Dabei ist ein Schalldruckdetektor ein besonders gut geeignetes Mittel. Der Schalldruckdetektor kann insbesondere ein piezoelektrischer Balken sein. Ein piezoelektrischer Balken ist vorzugweise eine schwingfähige Struktur, insbesondere in Form eines Biegebalkens, welcher ein piezoelektrisches Material, z. B. in Form eines Aktuators umfasst.

Dabei kann es bevorzugt sein, dass der Biegebalken passiv ist, das bedeutet bevorzugt, dass er durch die Schalldruckwellen zu einer Schwingung veranlasst wird. Diese erzeugen dabei wiederum durch die Verformung des piezoelektrischen Materials eine Spannung, welche auf dem piezoelektrischen Effekt beruht. Der (direkte) piezoelektrische Effekt beschreibt bevorzugt das Auftreten einer elektrischen Spannung und/oder eine Änderung der Impedanz an einem aus entsprechendem Material gefertigten Festkörper, wenn er elastisch verformt wird. Die Spannung kann z. B. durch geeignete Kontaktierung abgegriffen und durch eine entsprechende elektronische Schaltung ausgelesen werden.

Es kann ebenso bevorzugt sein, dass der Biegebalken aktiv ist, das bedeutet insbesondere, dass er aufgrund des inversen piezoelektrischen Effekts zu einer Schwingung veranlasst wird. Der piezoelektrische Effekt beschreibt bevorzugt die Verformung eines Materials bei Anlegen einer elektrischen Spannung und/oder eines elektrischen Feldes, wodurch insbesondere durch das Material eine Kraft ausgeübt werden kann. Durch die Schalldruckwellen kann dabei bevorzugt eine Variation der Dämpfung des schwingenden Balkens verursacht werden, welche messbar ist, z. B. durch eine Änderung der Resonanzfrequenz des schwingenden Balkens.

Ein passiv aufgrund von Schalldruckwellen schwingender Balken kann bevorzugt ebenso ausgelesen werden, z. B. durch kapazitive, magnetische und/oder piezoresistive Methoden. Hierbei können die Schwingungen eine elektrisch auslesbare Veränderung erzeugen, z. B. basierend auf einem sich ändernden magnetischen Fluss durch einen mitschwingenden Magneten, durch eine sich ändernde Kapazität zwischen einer schwingenden und einer feststehenden Elektrode und/oder durch einen sich ändernden elektrischen Widerstand in einem piezoresistiven Material.

Ein Mikrofon umfasst vorzugsweise eine schwingfähig gelagerte Membran, welche durch Schalldruckwellen zu Schwingungen angeregt wird, welche wiederum elektrisch auslesbar ist, ähnlich dem vorstehend beschriebenen Balken. Dabei können ebenfalls kapazitive, piezoelektrische und/oder piezoresistive Messmethoden der Schwingungsauslegung zur Anwendung kommen.

Bevorzugt kann ebenso ein optisches Mikrofon zur Anwendung kommen, wobei dies Schwingungen vorzugsweise durch Reflektion z. B. eines Laserstrahls auf der Membran in ein optisches Signal umgewandelt werden kann, welches z. B. in einer interferometrischen Anordnung ausgelesen wird.

In einer weiteren bevorzugten Ausführungsform der Erfindung ist der MEMS-Sensor ein kapazitives Mikrofon, umfassend eine MEMS-Membran als Elektrode sowie eine Gegenelektrode und wobei die MEMS-Membran bevorzugt eine maximale Ausdehnung in mindestens eine Richtung aufweist von 100 µm bis 1500 µm, insbesondere 200 bis 1000 µm.

Aufgrund der bevorzugt fehlenden mechanischen Komponenten sind MEMS-Sensoren dieser Ausführungsformen besonders einfach und kompakt herstellbar und dabei sehr robust.

In einem weiteren Aspekt und gemäß Anspruch 7 betrifft die Erfindung einen photoakustischen Gassensor zur Detektion von Aerosolpartikeln in einer Umgebungsluft mittels des beschriebenen Verfahrens.

Der durchschnittliche Fachmann erkennt, dass technische Merkmale, Definitionen und Vorteile bevorzugter Ausführungsformen des erfindungsgemäßen Verfahrens zur Detektion von Aerosolen in der Umgebungsluft auch für den photoakustischen Gassensors gelten und umgekehrt.

Der photoakustische Gassensor umfasst eine Steuereinheit, welche dafür konfiguriert ist, den Emitter auf eine selektive Anregung und Detektion der Aerosolpartikel zu steuern, wobei eine Abstimmung der Wellenlänge der emittierten Strahlung auf das Absorptionsverhalten der zu detektierenden Aerosolpartikel erfolgt.

Die Steuereinheit ist bevorzugt eine Einheit, welche zum Empfang, Verarbeiten, Erzeugen und/oder Senden von Steuerungssignalen bevorzugt von Messdaten, geeignet und konfiguriert ist. Die Steuereinheit umfasst bevorzugt zu diesem Zweck einen Prozessor, beispielsweise einen Mikroprozessor. Es können auch andere integrierte Schaltungen, welche in der digitalen Elektronik zur Steuerung verwendet werden, zum Einsatz kommen. Eine Steuereinheit, insbesondere in Form einer in den Emitter integrierten Steuerung, ist sehr kompakt und einfach zu handhaben.

Die Steuereinheit weist für die Eingabe bevorzugt eine geeignete Schnittstelle zur Verbindung bspw. mit einem Computer auf. Es kann ebenso gewünscht sein, dass über diese Schnittstelle auch Daten von der Steuerung an ein Eingabegerät übertragbar sind, wie beispielsweise die Modulationsfrequenz, eine oder mehrere Wellenlängen zur selektiven Anregung oder andere Statusinformationen.

Die Verwendung einer geeigneten Steuereinheit kann die gewünschte Verwendung des Spektrometers erheblich vereinfachen. Beispielsweise können geeignete Spektroskopie-Signale an einem PC gestaltet werden. Über ein Eingabemodul werden die gewünschten Signale an die Steuereinheit übertragen. Von dieser werden Ansteuerungssignale erzeugt, welche eine selektive Anregung Aerosolpartikel gewährleisten, wobei eine Abstimmung der Wellenlänge der emittierten Strahlung auf das Absorptionsverhalten der zu detektierenden Aerosolpartikel erfolgt.

Bevorzugt kann die Ansteuerung eines wellenlängenselektiven Emitters und/oder Filters auf Basis von Referenzdaten erfolgen, welche den spektralen Fingerabdruck der zu detektierenden Aerosolpartikel reflektieren. Die Steuersignale können dabei vorzugsweise derart eingestellt werden, dass die Anregung an einer oder mehrerer Wellenlängen erfolgt, bei denen für das zu detektierende Aerosolpartikel ein ausgeprägtes PAS-Signal zu erwarten ist.

In einem bevorzugten Ausführungsform umfasst der photoakustische Gassensor eine Datenverarbeitungseinheit, welche dafür konfiguriert ist, auf Basis einer Auswertung von Messergebnissen zu den erzeugten Schalldruckwellen eine Feststellung über die Präsenz und/oder Konzentration der Aerosolpartikeln in der Umgebungsluft zu treffen.

Die Datenverarbeitungseinheit ist bevorzugt eine Einheit, welche zum Empfang, Senden, Speichern und/oder Verarbeiten von Daten, bevorzugt von Messdaten, geeignet und konfiguriert ist. Die Datenverarbeitungseinheit umfasst bevorzugt einen integrierten Schaltkreis, ein Prozessor, ein Prozessorchip, Mikroprozessor oder Mikrokontroller zur Verarbeitung von Daten, sowie einen Datenspeicher, beispielsweise eine Festplatte, einen *random access memory* (RAM), einen *read-only memory* (ROM) oder auch einen *flash memory* zur Speicherung der Daten.

Zur Durchführung der Auswertung von Messergebnissen zu den erzeugten Schalldruckwellen und Feststellung über die Präsenz und/oder Konzentration der Aerosolpartikeln in der Umgebungsluft kann auf der Datenverarbeitungsvorrichtung bevorzugt ein Computerprogramm gespeichert vorliegen, welches Befehle umfasst, die genannten Schritte auszuführen.

Die Datenverarbeitungseinheit und Steuereinheit können bevorzugt auf denselben Prozessor zurückgreifen.

Besonders liegen auf der Datenverarbeitungseinheit bevorzugt Referenzdaten vor. Referenzdaten beziehen sich bevorzugt auf alle Daten, welche eine Feststellung der Präsenz und/oder Konzentration der Aerosolpartikel in der Umgebungsluft erlauben.

Wie obig erläutert können derartige Referenzdaten bevorzugt anhand von Kalibrationsmessungen erhalten werden, bei denen der photoakustische Gassensor in ein kontrolliertes Volumen mit bekannter Konzentration des zu detektierenden Aerosolpartikel gebracht wird. Durch eine PAS bei einer oder mehrere Wellenlängen unter derart kontrollierten Bedingung, können Referenzwerte für ein charakteristische PAS Spektrum zu detektierenden Aerosolpartikel erhalten werden, wobei vorzugweise die Amplitude der PAS Signale an den selektiven Wellenlängen mit der Konzentration der Aerosolpartikel korreliert.

Durch einen Vergleich der Messdaten mit den Referenzdaten bzw. Referenzwerten kann vorteilhaft festgestellt werden, ob die zu detektierenden Aerosolpartikel sich in der Umgebungsluft befinden und ggf. in welcher Konzentration.

Im Kontext der Erfindung meint die Feststellung einer Präsenz bevorzugt eine Aussage darüber ob, die Messdaten auf eine erhöhte Wahrscheinlichkeit für das Vorliegen von Aerosolpartikeln im Analysevolumen hinweisen. Bevorzugt ist dies gegeben bei Messung eines erhöhten PAS-Signales an einer oder mehrerer der selektiven Wellenlängen im Vergleich zu Referenzdaten unter Bedingungen in denen keine oder zumindest keine messbaren Aerosolpartikel in der Umgebungsluft vorliegen. Eine Konzentration der Aerosolpartikel meint bevorzugt eine quantitative Aussage über die Menge (z.B. Anzahl oder Masse) an Aerosolpartikeln für ein Volumen der Umgebungsluft. Dies kann, muss aber keine Aussage über absolute Werte der Konzentration beinhalten, sondern kann auch sich auf eine relative Konzentration gegenüber Referenzkonzentrationen beziehen.

In bevorzugten Ausführungsformen kann der photoakustischer Gassensor einen Signalgeber umfasst, wobei die Datenverarbeitungseinrichtung dafür konfiguriert ist, mittels des Signalgebers ein Warnsignal zu erzeugen, falls die bestimmte Konzentration der Aerosolpartikeln in der Umgebungsluft einen vorbestimmten Grenzwert überschreitet.

Der Signalgeber zur Ausgabe eines Warnsignals kann einen Lautsprecher oder eine optische Anzeige umfassen, um eine unmittelbare Warnung am photoakustischen Gassensor zu erzeugen. Der Signalsender kann aber auch das Warnsignal als ein digitales oder analoges elektrisches Signal an eine zentrale Datenverarbeitungseinheit weiterleiten, sodass auf Basis des Warnsignales weitere geeignete Schutzmaßnahmen ausgelöst werden können.

### Detaillierte Beschreibung

Im Folgenden soll die Erfindung an Hand von Beispielen und Figuren näher erläutert werden, ohne auf diese beschränkt zu sein.

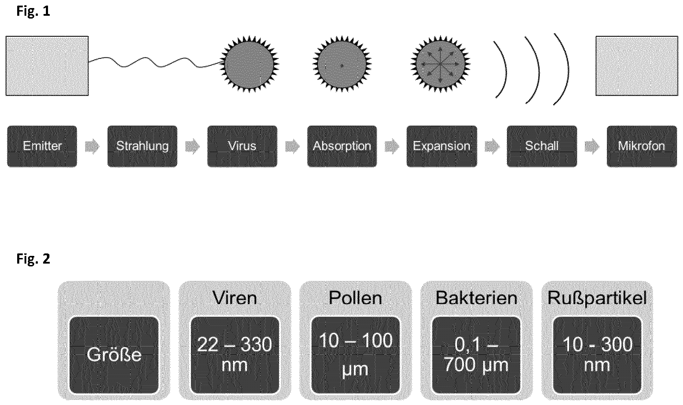

Figur 1 ist eine schematische Illustration einer bevorzugten Ausführungsform des Verfahrens zur Detektion von Aerosolpartikeln in einer Umgebungsluft, am Beispiel eines Virus.

Mittels eines modulierbaren Emitters wird elektromagnetische Strahlung erzeugt, insbesondere im infraroten, sichtbaren oder ultravioletten Wellenlängenbereich. Der Emitter ist dabei bevorzugt so angeordnet und konfiguriert, dass die von dem Emitter emittierte Strahlung im Wesentlichen auf das Analysevolumen trifft. Erfolgt die modulierte Bestrahlung mit einer Wellenlänge, welche dem Absorptionsspektrum eines sich in dem Analysevolumen befindlichen Virus entspricht, findet eine modulierte Absorption statt, die zu Erwärmungs- und Abkühlungsprozessen führt, deren Zeitskalen die Modulationsfrequenz der Strahlung widerspiegeln. So kann insbesondere die Proteinhülle von Viren die elektromagnetische Strahlung absorbieren und zu einer Expansion führen.

Gemäß dem photoakustischen Effekt führen die Erwärmungs- und Abkühlungsprozesse zu Expansionen und Kontraktionen von Bestandteilen des Virus (insbesondere den Proteinen der Proteinhülle) oder des gesamten Virus wodurch es zur Ausbildung von Schalldruckwellen mit im Wesentlichen der Modulationsfrequenz kommt. Die Schalldruckwellen lassen sich durch einen Schalldetektor, z.B. ein Mikrofon messen. Die Leistung der Schallwellen ist dabei vorzugsweise direkt proportional zur Konzentration der Viren im Analysevolumen.

Fig. 2 illustriert die unterschiedlichen Größen und Arten von Aerosolpartikeln, welche vorteilhaft mit dem erfindungsgemäßen Verfahren detektierbar sind, wobei die Detektion von Rußpartikeln nicht beansprucht ist.

### LITERATUR

Tobias, H. Bioaerosol mass spectrometry for rapid detection of individual airborne Mycobacterium tuberculosis H37Ra particles. Appl. Environ. Microbiol. 71, 6086-6095 (2005).
Fernstrom A. et al, Aerobiology and Its Role in the Transmission of Infectious Diseases Journal of Pathogens Volume 2013, Article ID 493960, 13 pages.
Senguptaa, A., Brarb, N. & Davis, E. J. Bioaerosol detection and characterization by surface-enhanced Raman spectroscopy. J. Colloid Interface Sci. 309, 36-43 (2007).
Schafer, M. P. et al. 1999. Detection and characterization of airborne Mycobacterium tuberculosis H37Ra particles, a surrogate for airborne pathogenic M. tuberculosis. Aerosol Sci. Technol. 30:161-173
Park, Kyu-Tae et al. Detection of airborne viruses using electro-aerodynamic deposition and a field-effect transistor, Scientific Reports | 5:17462 | DOI: 10.1038/srep17462.
Huffman et al Real-time sensing of bioaerosols: Review and current perspectives AEROSOL SCIENCE AND TECHNOLOGY 2020, VOL. 54, NO. 5, 465-495.
MARSH, D.G. (1975). Allergens and the genetics of allergy; in M. Sela (ed), The Antigens, Vol. 3, pp 271-359. (Academic Press Inc., London, New York).

## Patentansprüche

1. Verfahren zur Detektion von Aerosolpartikeln in einer Umgebungsluft, umfassend
a. Bereitstellung eines photoakustischen Gassensors, umfassend
- einen modulierbaren Emitter,
- ein Analysevolumen, welches in Fluidkommunikation mit der Umgebungsluft steht, wobei das Analysevolumen ein offenes System darstellt, welches eine oder mehrere Öffnungen aufweist, sodass die Umgebungsluft umfassend Aerosolpartikel in das Analysevolumen einströmen oder diffundieren kann
- einen MEMS-Sensor zur Detektion von Schalldruckwellen,
wobei das Analysevolumen im Strahlengang des Emitters vorliegt, sodass der Emitter mittels modulierbarer Strahlung Aerosolpartikel in dem Analysevolumen zur Ausbildung von Schalldruckwellen anregen kann, welche mit Hilfe des Sensors detektierbar sind,
b. Bestrahlung des Analysevolumen mit einer mit einer Modulationsfrequenz modulierten Strahlung zur Erzeugung von Schalldruckwellen
c. Messung der erzeugten Schalldruckwellen mittels des Sensors
d. Feststellung der Präsenz und/oder Konzentration der Aerosolpartikel in der Umgebungsluft anhand der Messergebnisse
**dadurch gekennzeichnet, dass**
es sich bei den Aerosolpartikeln um Bioaerosole handelt und wobei durch eine Steuereinheit eine selektive Anregung und Detektion der Aerosolpartikel durch eine Abstimmung der Wellenlänge der emittierten Strahlung auf das Absorptionsverhalten der Aerosolpartikel erfolgt.

2. Verfahren gemäß einem der vorherigen Ansprüche
**dadurch gekennzeichnet, dass**
es sich bei den Aerosolpartikeln um Pollen, Sporen, Bakterien oder Viren handelt.

3. Verfahren gemäß einem der vorherigen Ansprüche
**dadurch gekennzeichnet, dass**
es sich bei den Aerosolpartikeln bevorzugt um Pollen, Sporen, Bakterien oder Viren handelt, wobei die eine oder mehreren Wellenlängen des Emitters zur selektiven Anregung bevorzugt aus einem Bereich zwischen 170 nm und 1000 nm gewählt werden.

4. Verfahren gemäß einem der vorherigen Ansprüche
**dadurch gekennzeichnet, dass**
der modulierbare Emitter eine wellenlängenselektive Abstrahlung erlaubt und/oder im Strahlengang zwischen dem Emitter und dem Analysevolumen ein wellenlängenselektiver Filter, beispielsweise eine Fabry-Perot-Filter, vorliegt.

5. Verfahren gemäß einem der vorherigen Ansprüche
**dadurch gekennzeichnet, dass**
der modulierbare Emitter ein Infrarotemitter oder ein UV-Emitter ist.

6. Verfahren gemäß einem der vorherigen Ansprüche
**dadurch gekennzeichnet, dass**
der MEMS-Sensor ein Schalldruckdetektor ist, wobei der Schalldruckdetektor bevorzugt einen kapazitiv oder optisch auslesbaren, piezoelektrischen, piezoresistiven und/oder magnetischen Balken und/oder ein kapazitives, piezoelektrischen, piezoresistives und/oder optisches Mikrofon umfasst.

7. Photoakustischer Gassensor zur Detektion von Aerosolpartikeln in einer Umgebungsluft mittels einem Verfahren gemäß einem der vorherigen Ansprüche umfassend
- einen modulierbaren Emitter,
- ein Analysevolumen, welches in Fluidkommunikation mit der Umgebungsluft steht, wobei das Analysevolumen ein offenes System darstellt, welches eine oder mehrere Öffnungen aufweist, sodass die Umgebungsluft umfassend Aerosolpartikel in das Analysevolumen einströmen oder diffundieren kann
- einen MEMS-Sensor zur Detektion von Schalldruckwellen,
wobei das Analysevolumen im Strahlengang des Emitters vorliegt, sodass der Emitter mittels modulierbarer Strahlung Aerosolpartikeln in dem Analysevolumen zur Ausbildung von Schalldruckwellen anregen kann, welche mit Hilfe des Sensors detektierbar sind und wobei es sich bei den Aerosolpartikeln um Bioaerosole handelt und wobei der photoakustische Gassensor eine Steuereinheit umfasst, welche dafür konfiguriert ist den Emitter auf eine selektive Anregung und Detektion der Aerosolpartikel zu steuern, wobei eine Abstimmung der Wellenlänge der emittierten Strahlung auf das Absorptionsverhalten der zu detektierenden Aerosolpartikel erfolgt.

8. Photoakustischer Gassensor gemäß dem vorherigen Anspruch
**dadurch gekennzeichnet, dass**
der photoakustische Gassensor eine Datenverarbeitungseinheit umfasst, welcher dafür konfiguriert ist, auf Basis einer Auswertung von Messergebnissen zu den erzeugten Schalldruckwellen eine Feststellung über die Präsenz und/oder Konzentration der Aerosolpartikeln in der Umgebungsluft zu treffen.

9. Photoakustischer Gassensor gemäß einem der vorherigen Ansprüche 7 oder 8
**dadurch gekennzeichnet, dass**
der photoakustische Gassensor einen Signalgeber umfasst, wobei die Datenverarbeitungseinrichtung dafür konfiguriert ist, mittels des Signalgebers ein Warnsignal zu erzeugen, falls die festgestellte Konzentration der Aerosolpartikeln in der Umgebungsluft einen vorbestimmten Grenzwert überschreitet.

## Claims

1. Method for the detection of aerosol particles in ambient air, comprising
a. Providing a photoacoustic gas sensor comprising.
- a modulable emitter,
- an analysis volume which is in fluid communication with the ambient air, wherein the analysis volume is an open system having one or more openings such that ambient air comprising aerosol particles can flow or diffuse into the analysis volume,
- a MEMS sensor for the detection of sound pressure waves,
wherein the analysis volume is present in the beam path of the emitter such that the emitter can use modulable radiation to excite aerosol particles in the analysis volume to form sound pressure waves which are detectable by means of the sensor,
b. Irradiating the analysis volume with radiation modulated with a modulation frequency to generate sound pressure waves
c. Measuring the generated sound pressure waves by means of the sensor
d. Determining the presence and/or concentration of aerosol particles in the ambient air based on the measurement results
**characterized in that**
the aerosol particles are bioaerosols and wherein by means of a control unit a selective excitation and detection of the aerosol particles is performed by tuning the wavelength of the emitted radiation to the absorption behavior of the aerosol particles.

2. Method according to one of the preceding claims
**characterized in that**
the aerosol particles are pollen, spores, bacteria or viruses.

3. Method according to one of the preceding claims
**characterized in that**
the aerosol particles are preferably pollen, spores, bacteria or viruses, wherein the one or more wavelengths of the emitter for selective excitation are preferably selected from a range between 170 nm and 1000 nm.

4. Method according to one of the preceding claims
**characterized in that**
the modulable emitter permits wavelength-selective radiation and/or a wavelength-selective filter, for example a Fabry-Perot filter, is present in the beam path between the emitter and the analysis volume.

5. Method according to one of the previous claims
**characterized in that**
the modulable emitter is an infrared emitter or a UV emitter.

6. Method according to one of the preceding claims
**characterized in that**
the MEMS sensor is a sound pressure detector, the sound pressure detector preferably comprising a capacitively or optically readable piezoelectric, piezoresistive and/or magnetic beam and/or a capacitive, piezoelectric, piezoresistive and/or optical microphone.

7. Photoacoustic gas sensor for detecting aerosol particles in ambient air by a method according to any one of the preceding claims, comprising
- a modulable emitter,
- an analysis volume which is in fluid communication with the ambient air, wherein the analysis volume is an open system having one or more openings such that ambient air comprising aerosol particles can flow or diffuse into the analysis volume,
- a MEMS sensor for the detection of sound pressure waves,
wherein the analysis volume is present in the beam path of the emitter such that the emitter can use modulable radiation to excite aerosol particles in the analysis volume to form sound pressure waves which are detectable by means of the sensor,
wherein the aerosol particles are bioaerosols and wherein the photoacoustic gas sensor comprises a control unit configured to control the emitter to selectively excite and detect the aerosol particles, wherein the wavelength of the emitted radiation is tuned to the absorption behavior of the aerosol particles to be detected.

8. Photoacoustic gas sensor according to the preceding claims
**characterized in that**
the photoacoustic gas sensor comprises
a data processing unit configured to make a determination about the presence and/or concentration of the aerosol particles in the ambient air based on an evaluation of measurement results for the generated sound pressure waves.

9. Photoacoustic gas sensor according to any one of the preceding claims 7 or 8.
**characterized in that**
the photoacoustic gas sensor comprises a signal generator, the data processing means being configured to generate a warning signal by means of the signal generator if the detected concentration of the aerosol particles in the ambient air exceeds a predetermined threshold.

## Revendications

1. Procédé de détection de particules d'aérosol dans un air ambiant, comprenant
a. la fourniture d'un capteur de gaz photoacoustique, comprenant
- un émetteur modulable,
- un volume d'analyse qui est en communication fluidique avec l'air ambiant, dans lequel le volume d'analyse représente un système ouvert qui présente une ou plusieurs ouvertures de sorte que l'air ambiant comprenant des particules d'aérosol peut s'écouler ou se diffuser dans le volume d'analyse
- un capteur MEMS de détection d'ondes de pression acoustique,
dans lequel le volume d'analyse est présent dans le trajet du faisceau de l'émetteur, de sorte que l'émetteur peut stimuler les particules d'aérosol dans le volume d'analyse au moyen d'un rayonnement modulé afin de former des ondes de pression acoustique, qui peuvent être détectées à l'aide du capteur,
b. l'irradiation du volume d'analyse avec un rayonnement modulé avec une fréquence de modulation pour générer des ondes de pression acoustique
c. la mesure des ondes de pression acoustique générées à l'aide du capteur
d. la détermination de la présence et/ou de la concentration de particules d'aérosol dans l'air ambiant sur la base des résultats de mesure
**caractérisé en ce que**
les particules d'aérosol sont des bioaérosols et dans lequel une unité de commande excite et détecte sélectivement les particules d'aérosol en faisant correspondre la longueur d'onde du rayonnement émis au comportement d'absorption des particules d'aérosol.

2. Procédé selon l'une des revendications précédentes
**caractérisé en ce que**
les particules d'aérosol sont du pollen, des spores, des bactéries ou des virus.

3. Procédé selon l'une des revendications précédentes
**caractérisé en ce que**
les particules d'aérosol sont de préférence du pollen, des spores, des bactéries ou des virus, dans lequel les une ou plusieurs longueurs d'onde de l'émetteur pour une excitation sélective sont de préférence sélectionnées dans une plage comprise entre 170 nm et 1 000 nm.

4. Procédé selon l'une des revendications précédentes
**caractérisé en ce que**
l'émetteur modulable permet un rayonnement sélectif en longueur d'onde et/ou un filtre sélectif en longueur d'onde, par exemple un filtre Fabry-Pérot, est présent dans le trajet du faisceau entre l'émetteur et le volume d'analyse.

5. Procédé selon l'une des revendications précédentes
**caractérisé en ce que**
l'émetteur modulable est un émetteur infrarouge ou un émetteur UV.

6. Procédé selon l'une des revendications précédentes
**caractérisé en ce que**
le capteur MEMS est un détecteur de pression acoustique, dans lequel le détecteur de pression acoustique comprend de préférence une barre piézoélectrique, piézorésistive et/ou magnétique à lecture capacitive ou optique et/ou un microphone capacitif, piézoélectrique, piézorésistif et/ou optique.

7. Capteur de gaz photoacoustique de détection de particules d'aérosol dans un air ambiant au moyen d'un procédé selon l'une des revendications précédentes, comprenant
- un émetteur modulable,
- un volume d'analyse qui est en communication fluidique avec l'air ambiant, dans lequel le volume d'analyse représente un système ouvert qui présente une ou plusieurs ouvertures de sorte que l'air ambiant comprenant des particules d'aérosol peut s'écouler ou se diffuser dans le volume d'analyse
- un capteur MEMS de détection d'ondes de pression acoustique,
dans lequel le volume d'analyse est présent dans le trajet de faisceau de l'émetteur, de sorte que l'émetteur peut utiliser un rayonnement modulé pour stimuler les particules d'aérosol dans le volume d'analyse afin de former des ondes de pression acoustique, qui peuvent être détectées à l'aide du capteur et dans lequel les particules d'aérosol sont des bioaérosols et dans lequel le capteur de gaz photoacoustique comprend une unité de commande qui est configurée pour commander l'émetteur sur une excitation sélective et une détection des particules d'aérosol, dans lequel la longueur d'onde du rayonnement émis est mise en correspondance avec le comportement d'absorption des particules d'aérosol à détecter.

8. Capteur de gaz photoacoustique selon la revendication précédente
**caractérisé en ce que**
le capteur de gaz photoacoustique comprend une unité de traitement de données qui est configurée pour effectuer une détermination concernant la présence et/ou la concentration des particules d'aérosol dans l'air ambiant sur la base d'une évaluation des résultats de mesure des ondes de pression acoustique générées.

9. Capteur de gaz photoacoustique selon l'une des revendications précédentes 7 ou 8
**caractérisé en ce que**
le capteur de gaz photoacoustique comprend un générateur de signal, dans lequel le dispositif de traitement de données est configuré pour générer un signal d'avertissement au moyen du générateur de signal si la concentration déterminée des particules d'aérosol dans l'air ambiant dépasse une valeur limite prédéterminée.
